# EUROPEAN PATENT SPECIFICATION

(11) **EP 3 484 453 B1**
(45) Date of publication and mention of the grant of the patent: **04.12.2019**
(21) Application number: 18706628.7
(22) Date of filing: 17.01.2018
(51) Int. Cl.: A61K 9/46, A61K 47/02, A61K 47/12, A61K 9/16, A61K 9/20, A61P 25/04, A61P 29/00

(54) **EFFERVESCENT COMPOSITION CONTAINING METAMIZOLE SODIUM MONOHYDRATE AND METHOD OF PREPARING SAME**
BRAUSE ZUSAMMENSETZUNG ENTHALTEND METAMIZOL NATRIUM MONOHYDRAT UND DEREN HERSTELLUNG
COMPOSITION EFFERVESCENT COMPRENNANT DE METAMIZOL MONOHYDRATE DE SODIUM ET METHODE DE PREPARATION

(30) Priority: 19.01.2017 BG 11244317
(43) Date of publication of application: 22.05.2019
(73) Proprietor: Adipharm, Ead, 1700 Sofia (BG)
(72) Inventor: HALED HOMSY, Sahtura Abdulsalam, 1618 Sofia (BG); LAZAROVA, Radoevska Nadka, 1505 Sofia (BG)
(74) Representative: Valcheva, Emiliya Nikolova
(86) International application number: PCT/BG2018/000003
(87) International publication number: WO 2018/132880

(56) References cited:
- WO-A1-00/47189
- WO-A1-2014/194872

## Description

### Field of invention

The invention relates to an effervescent composition, containing metamizole sodium monohydrate and to a method of preparing same that finds application in pharmaceutical practice and medicine.

### Background of the invention

Effervescent compositions are known and are used in medicine for many years (Pharmaceutical dosage Forms: Tablets Vol. I, A. Lieberman. ed., 1989). Effervescent dosage forms, apart from being active ingredients, also contain an effervescent pair. The conventional effervescent pair is defined as a combination of a carbonate salt and an organic acid. Typical carbonate salts are hydrogen carbonates or carbonates of sodium, potassium, magnesium and calcium, and the most frequently used organic acids are citric acid, fumaric acid, vinegar acid, adipic acid, amber acid and malic acid. Therapeutic effect is achieved much faster with effervescent pharmaceutical compositions than with the conventional oral dosage forms. They are received easily by the patients, even by those that have problems with swallowing tablets and capsules.

Metamizole is an analgesic that is known and used in medical practice. It also has an antipyretic and spasmolytic effect. Metamizole, however, is an unstable chemical compound and poses problems in including it in pharmaceutical compositions. Literature data reveal that metamizole disintegrates in solid and liquid dosage forms as a result of hydrolytic and oxidation processes (Fabre, H.; Eddine, N. H.; Bressolle, F.; Mondrou, B.; Analyst 1982, 107, 61). The influence of the physicochemical factors on the kinetics of hydrolysis of metamizole sodium in water solutions is also well studied and described by H. Ergun et al. (Characterization of the role of physicochemical factors on the hydrolysis of dipyrone, Journal of Pharmaceutical and Biomedical Analysis, Volume 35, Issue 3, 28 May 2004, pages 479-487). Using HPLC methods of analysis, it is established that this process is influenced by concentration, temperature and pH.

Available on the pharmaceutical market is a product in the form of an effervescent tablet Novalgin® akut, containing 500 mg of metamizole sodium monohydrate, an effervescent mixture, consisting of citric acid, sodium hydrogen carbonate and sodium carbonate, and excipients: saccharin sodium, sodium cyclamate and macrogol 6000. In its Summary of product characteristics (SPmC), in accordance with the requirements of EMA, FDA and WHO, contents of 8.72 mmol of sodium in a tablet and an admissible daily administration of 3 to 5 tablets is claimed.

The maximum recommended daily dose of sodium, determined by EMA, FDA and WHO is not higher than 17 mmol, in order to avoid the risk of blood pressure elevation and development of arterial hypertonia, and, according to certain investigations, stomach cancer. According to the requirements of these organizations, this medicinal form is designated with a warning caption - a product with "high sodium content". (Guidelines Medicinal products for human use Safety, environment and information, Brussels, ENTR/F2/BL D(2003))

European Patent EP1150660 describes a stable pharmaceutical composition of metamizole in a formulation of an effervescent powder, tablets or granules. The composition contains from 200 to 1000 mg of metamizole, preferably, metamizole sodium monohydrate, and an effervescent mixture in a quantity higher than 57 wt.%, that includes sodium carbonate and sodium hydrogen carbonate in combination with citric acid or mixture of citric and ascorbinic acid, and also other excipients, such as saccharin sodium, sodium cyclamate, macrogol 6000 and flavours. pH of the water solution of this composition, prepared for use, is from 3 to 6.5. The allegation that under those conditions Metamizole in the solution is stable and does not hydrolyse within 1 hour of dissolution is explained by the fact that the solution in this time's period remains colorless and does not turn yellow.

According to that patent, the effervescent pharmaceutical composition is obtained by dry mixing of the components and direct tableting.

A disadvantage of this effervescent composition is the high sodium content, 21.3 mmol Na per tablet, that, at a daily administration of 4 tablets, increases to 85.2 mmol Na, and the water solution prepared for use has unproven stability of the active ingredient in terms of the hydrolysis product of Metamizole - impurity C (4-methylaminoantipyrine/MAA).

An object of the present invention is to create an effervescent composition and a method of preparing same that will ensure an effervescent pharmaceutical form with low sodium content, stable during storage, with a high rate of water dissolution without stirring and whose water solution has good organoleptic properties and good quality in terms of impurity C content (4-methylaminoantipyrine/MAA), the hydrolysis product of metamizole sodium.

### Disclosure of the invention

The effervescent composition, according to the invention, contains metamizole sodium monohydrate and an effervescent mixture, comprising water-free sodium carbonate and water-free citric acid, as well as excipients: water-free magnesium citrate, saccharose for direct tableting, water-free colloidal silicon dioxide, sodium cyclamate, saccharin sodium and a flavor. The components of the effervescent composition are in the following quantitative proportions in wt.%: from 29,41 to 33,33 metamizole sodium monohydrate, from 8,47 to 9.6 water-free sodium carbonate, from 11,76 to 13.33 water-free citric acid, from 3.33 to 5,88 water-free magnesium citrate, from 35.60 to 38,47 saccharose for direct tableting, from 0.27 to 0,41 water-free colloidal silicon dioxide, from 2,35 to 2.67 sodium cyclamate, from 0.20 to 0,29 saccharin sodium and from 1.67 to 2,94 a flavour.

The effercescent composition, in accordance with the invention, is in the form of powder or granules and is suitable for dosing in sachets for one-off administration. It is soluble in water in less than two minutes without stirring, resulting in a transparent, ready-to-use solution with pH 5.8 ÷ 6.2.

Unlike effervescent tablets, in which the processes of effervescent reaction and dissolution of the components in water take place simultaneously, in the effervescent composition, according to the invention, which has a low content of effervescent pair, first is observed a process of dissolution of the components of the composition in water and after that, the effervescent reaction between the water-free citric acid and the water-free sodium carbonate takes place.

The saccharose for direct tableting is a diluting and sweetening substance in the effervescent composition of metamizole sodium monohydrate, according to the invention, but it is also a solubilizing component of the composition and has water solubility rate, comparable to that of the rest of components of the effervescent composition.

The effervescent powderlike mixture, according to the invention, is prepared by mixing the pre-weighed quantities of metamizole sodium monohydrate, saccharose for direct tableting, water-free sodium carbonate and water-free colloidal silicon dioxide. The mixture is homogenized for 30 min. The water-free citric acid and the other substances included in the composition are added to the homogenized mixture. The mixture is sieved through a sieve and is dosed in sachets of three-layered foil.

To obtain the effervescent mixture in accordance with the invention, when the bulk density of metamizole sodium monohydrate is lower than 0.300 g/ml, the method of dry granulation of the active ingredient of metamizole sodium monohydrate in a mixture with water-free sodium carbonate and part of the total quantity of water-free colloidal silicon dioxide is implemented. The dry mixture is fed for granulation in a machine "Roller compactor". The obtained granules are mixed with the other components of the composition. The finished mixture is dosed in sachets of three-layered foil.

The advantage of the effervescent composition and the method of preparing same, according to the invention, is that the obtained effervescent medicinal form has low sodium content, is stable during storage, has high water solubility rate without stirring, and the obtained water solution has good organoleptic properties and good quality in terms of the content of the impurity C (4-methylaminoantipyrine /MAA), the hydrolysis product of Metamizole sodium.

### Examples

The invention is illustrated by the following examples. They should not be considered as limiting the scope of present invention, but merely as being representative thereof:

### Example 1. To obtain an effervescent powder in a sachet, containing 500 mg of metamizole sodium monohydrate, the following components were used:

| **Component name** | **mg/in sachet** | **%** |
|---|---|---|
| Metamizole sodium monohydrate | 500 | 29.41 |
| Water-free citric acid | 200.0 | 11.76 |
| Water-free sodium carbonate | 144.0 | 8.47 |
| Compressible sugar* | 654.0 | 38.47 |
| Water-free magnesium citrate | 100.0 | 5.88 |
| Sodium cyclamate | 40.0 | 2.35 |
| Saccharin sodium | 5.0 | 0.29 |
| Powder-like lemon flavour | 50.0 | 2.94 |
| Aerosil® 200** | 7.0 | 0.41 |
| **Total** | **1700** | **100.0** |

| | | |
|---|---|---|
| * Compressible sugar is 98% saccharose, suitable for direct tableting, dried in advance at 105 °C for 4 hours. **Aerosil® 200 is a trademark of water-free colloidal silicon dioxide - product of Degussa GmbH. | | |

To obtain the effervescent powder, 0.500 kg of metamizole sodium monohydrate, 0.654 kg of saccharose (compressible sugar), 0.144 kg of water-free sodium carbonate and 0.007 kg of water-free Aerosil® 200 are mixed in a suitable diffusion mixer for 30 min. 0.200 kg of water-free citric acid and the remaining ingredients in the formulation are added to the obtained mixture. The mixture is homogenized for 15 min, and then it is sieved through a sieve with a clear opening of 1,25 mm. The finished mixture is dosed in sachets of three-layered foil with a packing machine at an average mass of 1.7 g of the dosed mixture in a sachet.

The effervescent powder, described in Example 1 was investigated for stability under monitoring conditions: 25 ± 2 °C and relative air humidity 60 ± 5% RH for 24 months. The results of the investigation are given in Table 1.

**Table 1.**

| Tests | Initial analysis | Test after 3 months | Test after 6 months | Test after 9 months | Test after 12 months | Test after 18 months | Test after 24 months |
|---|---|---|---|---|---|---|---|
| Appearance | Compliant | Compliant | Compliant | Compliant | Compliant | Compliant | Compliant |
| Identity of Metamizole sodium | Compliant | Compliant | Compliant | Compliant | Compliant | Compliant | Compliant |
| Mass of contents in a sachet, g | 1.718 | 1.718 | 1.720 | 1.721 | 1.721 | 1.721 | 1.722 |
| pH of solution | 5.94 | 5.93 | 5.95 | 5.94 | 5.96 | 5.94 | 5.95 |

| Contents of similar substances, % | | | | | | | |
|---|---|---|---|---|---|---|---|
| - Impurity C | 0.46 | 0.52 | 0.57 | 0.59 | 0.62 | 0.64 | 0.64 |
| - Other single impurity | 0.040 | 0.040 | 0.040 | 0.041 | 0.042 | 0.042 | 0.042 |
| - Total impurities | 0.500 | 0.560 | 0.610 | 0.631 | 0.662 | 0.682 | 0.682 |
| Contents of Metamizole sodium, mg | 496.4 | 496.3 | 496.1 | 495.8 | 495.8 | 495.6 | 495.2 |

| Microbiological quality: | | | | | | | |
|---|---|---|---|---|---|---|---|
| Total Aerobic Microbial Count (TAMC), CFU/g | < 10 | < 10 | < 10 | < 10 | < 10 | < 10 | < 10 |
| Total Yeast and Mold Count (TYMC), CFU/g | < 10 | < 10 | < 10 | < 10 | < 10 | < 10 | < 10 |
| Escherichia coli,/g | Absent | Absent | Absent | Absent | Absent | Absent | Absent |

### Example 2. To obtain an effervescent powder in a sachet, containing 1000 mg of metamizole sodium monohydrate, the following components were used:

| **Component name** | **mg/sachet** | **%** |
|---|---|---|
| Metamizole sodium monohydrate | 1000 | 33.33 |
| Water-free citric acid | 400.0 | 13.33 |
| Water-free sodium carbonate | 288.0 | 9.60 |
| Compressible sugar* | 1068.0 | 35.60 |
| Water-free magnesium citrate | 100.0 | 3.33 |
| Sodium cyclamate | 80.0 | 2.67 |
| Saccharin sodium | 6.0 | 0.20 |
| Powder-like lemon flavour | 50.0 | 1.67 |
| Aerosil® 200** | 8.0 | 0.27 |
| **Total** | **3000** | **100.0** |

| | | |
|---|---|---|
| * Compressible sugar is 98% saccharose, suitable for direct tableting, dried in advance at 105 °C for 4 hours. **Aerosil® 200 is a trademark of water-free colloidal silicon dioxide - product of Degussa GmbH. | | |

The effervescent powder is prepared by the method described in Example 1.

The results of the investigations for stability of the dosage form, obtained in accordance with Example 2, are presented in Table 2. Monitoring conditions are: 25 ± 2 °C, 60 ± 5 % RH for 24 months.

**Table 2.**

| Tests | Initial analysis | Test after 3 months | Test after 6 months | Test after 9 months | Test after 12 months | Test after 18 months | Test after 24 months |
|---|---|---|---|---|---|---|---|
| Appearance | Compliant | Compliant | Compliant | Compliant | Compliant | Compliant | Compliant |
| Identity of Metamizole sodium | Compliant | Compliant | Compliant | Compliant | Compliant | Compliant | Compliant |
| Mass of contents in a sachet, g | 3.019 | 3.020 | 3.020 | 3.021 | 3.022 | 3.022 | 3.023 |
| pH of solution | 5.86 | 5.88 | 5.88 | 5.89 | 5.91 | 5.91 | 5.92 |

| Contents of similar substances, % | | | | | | | |
|---|---|---|---|---|---|---|---|
| - Impurity C | 0.51 | 0.52 | 0.56 | 0.58 | 0.61 | 0.63 | 0.66 |
| - Other single impurity | 0.041 | 0.040 | 0.041 | 0.042 | 0.042 | 0.043 | 0.043 |
| - Total impurities | 0.551 | 0.560 | 0.601 | 0.622 | 0.652 | 0.673 | 0.703 |
| Contents of Metamizole sodium, mg | 1008.3 | 1008.1 | 1007.8 | 1007.8 | 1007.6 | 1007.4 | 1007.3 |

| Microbiological quality: | | | | | | | |
|---|---|---|---|---|---|---|---|
| Total Aerobic Microbial Count (TAMC), CFU/g | < 10 | < 10 | < 10 | < 10 | < 10 | < 10 | < 10 |
| Total Yeast and Mold Count | | | | | | | |
| (TYMC), CFU/g | < 10 | < 10 | < 10 | < 10 | < 10 | < 10 | < 10 |
| Escherichia coli,/g | Absent | Absent | Absent | Absent | Absent | Absent | Absent |

### Example 3. To obtain effervescent granules, containing 500 mg of metamizole sodium monohydrate in a sashet, the following components were used:

| **Component name** | **mg/sachet** | **wt. %** |
|---|---|---|
| Metamizole sodium monohydrate with bulk density lower than 0.300 g/ml, | 500 | 29.41 |
| Water-free citric acid | 200.0 | 11.76 |
| Water-free sodium carbonate | 144.0 | 8.47 |
| Compressible sugar* | 654.0 | 38.47 |
| Water-free magnesium citrate | 100.0 | 5.88 |
| Sodium cyclamate | 40.0 | 2.35 |
| Saccharin sodium | 5.0 | 0.29 |
| Powder-like lemon flavour | 50.0 | 2.94 |
| Aerosil® 200** | 7.0 | 0.41 |
| O 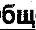 o | **1700** | **100.0** |

| | | |
|---|---|---|
| *Compressible sugar is 98% saccharose, suitable for direct tableting, dried in advance at 105 °C for 4 hours. **Aerosil® 200 is a trademark of water-free colloidal silicon dioxide - product of Degussa GmbH. | | |

In order to obtain dry granules of metamizole sodium monohydrate, roller compaction is implemented. Metamizole sodium monohydrate, water-free sodium carbonate and 57.14% of the total quantity of Aerosil 200 are mixed in a suitable diffusion mixer for 15 min. The dry mixture is fed for granulation in a roller compactor type RC 100x30 PHARMA, in the following set optimal parameters:
Screw Feeder - 25 rpm;
Roller velocity - 9 rpm;
Hydraulic pressure - 60 bar;
Granule sieve size - 1.25 mm.

In the final mixer, the obtained granules, compressible sugar, the remaining quantity of Aerosil 200 and the other ingredients in the formulation are mixed for 20 min. The finished mixture is dosed in sachets of three-layered foil of average mass of 1.7 g in a packing machine.

The results of the investigations for stability of the dosage form, obtained in accordance with Example 3, are given in Table 3. Monitoring conditions are: 25 ± 2 °C, 60 ± 5% RH for 24 months.

**Table 3.**

| Tests | Initial analysis | Test after 3 months | Test after 6 months | Test after 9 months | Test after 12 months | Test after 18 months | Test after 24 months |
|---|---|---|---|---|---|---|---|
| Appearance | Compliant | Compliant | Compliant | Compliant | Compliant | Compliant | Compliant |
| Identity of Metamizole sodium | Compliant | Compliant | Compliant | Compliant | Compliant | Compliant | Compliant |
| Mass of contents in a sachet, g | 1.716 | 1.717 | 1.722 | 1.722 | 1.723 | 1.723 | 1.724 |
| pH of solution | 5.88 | 5.88 | 5.89 | 5.90 | 5.89 | 5.90 | 5.90 |

| Contents of similar substances, % | | | | | | | |
|---|---|---|---|---|---|---|---|
| - Impurity C | 0.49 | 0.54 | 0.59 | 0.62 | 0.65 | 0.67 | 0.68 |
| - Other single impurity | 0.039 | 0.043 | 0.045 | 0.045 | 0.046 | 0.048 | 0.048 |
| - Total impurities | 0.529 | 0.583 | 0.635 | 0.665 | 0.696 | 0.718 | 0.728 |
| Contents of Metamizole sodium, mg | 502.3 | 501.8 | 501.6 | 501.6 | 501.4 | 501.2 | 501.2 |

| Microbiological quality: | | | | | | | |
|---|---|---|---|---|---|---|---|
| Total Aerobic Microbial Count (TAMC), CFU/g | < 10 | < 10 | < 10 | < 10 | < 10 | < 10 | < 10 |
| Total Yeast and Mold Count (TYMC), CFU/g | < 10 | < 10 | < 10 | < 10 | < 10 | < 10 | < 10 |
| Escherichia coli,/g | Absent | Absent | Absent | Absent | Absent | Absent | Absent |

### Example 4: Test results for solubility and assessment of the effervescent composition solution, described in Example 3, prepared for use.

Each sample-sachet is dissolve under the same conditions in 150 ml of water at a temperature of 20 °C. The test results are presented in Table 4, as follows:

**Table 4**

| **Tested parameters** | **Limit** | **Test results** |
|---|---|---|
| Dissolution time in 100 ml of water at a temperature of 20 °C | not more than 5 min | 1.5min |
| Appearance of the solution | limpid to opalescent | limpid solution, no sediment |
| pH of solution | from 5.3 to 6.5 | 5.84 |
| Organoleptic properties of the solution | pleasant, without explicit sweet and bitter taste | pleasant, without explicit sweet and bitter taste, with lemon flavour |

### Example 5. Analysis of the obtained peroral solution after dissolution of one sachet of effervescent powder, containing metamizole sodium monohydrate 500 mg of composition, described in Example 1.

In order to assess the suitability of the prepared peroral solution after dissolving one sachet in a certain quantity of water in terms of the claimed contents of metamizole sodium monohydrate in one 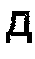 o3a - 500 mg, the obtained solution was analysed for the quantity of the active ingredient, as well as for the impurity contents, in certain intervals of time as of the time of dissolution.

Although the prepared peroral solution is intended for immediate administration, the solution was analysed at intervals of time of 0, 10, 15 min with the aim of assessing its quality - contents of metamizole sodium monohydrate and of its hydrolysis products and other impurities.

Evert sample-sachet is dissolved under the same conditions in 150 ml of water at a temperature of 20 °C. The temperature is maintained constant for 15 min and sampes for analysis are taken from the solution at certain intervals of time.

Each sample is tested three times. The results of the analysis are presented in Table 5:

**Table 5. Analysis of the solution after dissolution of one sachet of the product**

| Time, min | *Effervescent powder, containing metamizole sodium monohydrate* 500 mg | | | |
|---|---|---|---|---|
| | Metamizole sodium monohydrate, % | Impurity C (MAA), % | Other single impurity, % | Total impurities % |
| 0 | 100.24 | 0.52 | 0.04 | 0.56 |
| 10 | 97.83 | 1.88 | 0.11 | 1.99 |
| 15 | 96.76 | 2.87 | 0.14 | 3.01 |

The results show that the quality of the prepared water solution of effervescent powder, containing metamizole sodium monohydrate 500 mg, according to the invention, is preserved within 15 min of dissolution in terms of the claimed contents of the active substance metamizole sodium monohydrate in the product - 100 ± 5%.

The quantity of the C impurity (4-methylaminoantipyrine/MAA) in the solution within 10 min is around 2%, that corresponds to the value, established in the liquid dosage forms of metamizole sodium monohydrate.

The results obtained show that the solutions are suitable for peroral use up to 10 min after dissolution of the effervescent composition, according to the invention.

## Claims

1. Effervescent composition, containing metamizole sodium monohydrate and effervescent mixture, including sodium carbonate and citric acid, as well as excipients saccharin sodium and flavour, **characterized by** that, the sodium carbonate and the citric acid are in water-free form, the composition also containing water-free magnesium citrate, saccharose for direct tableting, water-free colloidal silicon dioxide and sodium cyclamate, and the quantitative ratios of the components of the composition in wt.% are, as follows: from 29,41 to 33,33 metamizole sodium monohydrate, from 8,47 to 9.6 water-free sodium carbonate, from 11,76 to 13.33 water-free citric acid, from 3.33 to 5,88 water-free magnesium citrate, from 35.60 to 38,47 saccharose for direct tableting, from 0.27 to 0,41 water-free colloidal silicon dioxide, from 2,35 to 2.67 sodium cyclamate, from 0.20 to 0,29 saccharin sodium and from 1.67 to 2,94 flavour.

2. Effervescent composition, containing metamizole sodium monohydrate according to claim 1, **characterized by** that it is in the form of effervescent powder.

3. Effervescent composition, containing metamizole sodium monohydrate as claimed in Claim 1, **characterized by** that it is in the form of effervescent granules.

4. Method of production of effervescent composition, according to any one of the claims from 1 to 3, **characterized by** that metamizole sodium monohydrate is mixed with water-free sodium carbonate and part of the water-free colloidal silicon dioxide, the dry mixture is fed for granulation by roller compaction, the granules obtained are mixed with water-free citric acid, water-free magnesium citrate, sodium cyclamate, saccharin sodium, flavour and with the remaining quantity of water-free colloidal silicon dioxide, and the granule mixture is sieved and dosed in sachets of three-layered foil.

## Patentansprüche

1. Brause zusammensetzung enthaltend Metamizol Natrium Monohydrat und eine Brausemischung enthaltend Natriumcarbonat und Zitronensäure sowie die Hilfsstoffe Saccharin-Natrium und Aroma, **dadurch gekennzeichnet, dass** Natriumcarbonat und Zitronensäure in wasserfreier Form vorliegen, die Zusammensetzung enthält auch wasserfreies Magnesiumcitrat, Saccharose zur direkten Tablettierung, kolloidales wasserfreies Siliciumdioxid und Natriumcyclamat und die Mengenverhältnisse der Komponenten der Zusammensetzung in Gewichtsprozente sind wie folgt: 29,41 bis 33,33 Metamizol Natrium Monohydrat, 8,47 bis 9.6 wasserfreies Natriumcarbonat, 11,76 bis 13.33 wasserfreie Zitronensäure, 3.33 bis 5,88 wasserfreies Magnesiumcitrat, 35.60 bis 38,47 Saccharose zur direkten Tablettierung, 0.27 bis 0,41 kolloidales wasserfreies Siliziumdioxid, 2,35 bis 2.67 Natriumcyclamat, 0.20 bis 0,29 Saccharin-Natrium und 1.67 bis 2,94 Aroma.

2. Brausezusammensetzung, enthaltend Metamizol Natrium Monohydrat nach Anspruch 1, **dadurch gekennzeichnet, dass** sie in der Form von Brausepulver ist.

3. Brausezusammensetzung, enthaltend Metamizol Natrium Monohydrat gem. Anspruch 1, **dadurch gekennzeichnet, dass** sie in der Form von Brausegranulat ist.

4. Verfahren zum Herstellen einer Brausezusammensetzung nach den Ansprüchen 1 und 3, **dadurch gekennzeichnet, dass** das Metamizol Natrium Monohydrat mit wasserfreiem Natriumcarbonat und einem Teil dem kolloidalen wasserfreien Siliziumdioxid gemischt wird, wobei die trockene Mischung zur Granulierung durch Walzenpressen zugeführt wird und die erhaltenen Granulate mit wasserfreier Zitronensäure, wasserfreiem Magnesiumcitrat, Natriumcyclamat, Saccharinnatrium, Aroma und der verbleibenden Menge an wasserfreiem kolloidalem Siliciumdioxid gemischt werden und die resultierende körnige Mischung gesiebt und in einem Beutel aus dreischichtiger Folie dosiert werden.

## Revendications

1. Composition effervescente comprenant du métamizole sodique monohydraté et un mélange effervescent comprenant du carbonate de sodium et de l'acide citrique, ainsi que des excipients saccharine sodique et arôme, **caractérisé en ce que** le carbonate de sodium et l'acide citrique sont sous forme anhydre, la composition contient également du magnésium anhydre citrate, saccharose pour la fabrication directe de comprimés, dioxyde de silicium colloïdal anhydre et cyclamate de sodium, et les rapports quantitatifs des composants de la composition en % en poids sont les suivants: de 29,41 à 33,33 de métamizole de sodium monohydraté, de 8,47 à 9,6 carbonate de sodium anhydre, de 11,76 à 13,33 d'acide citrique anhydre, de 3,33 à 5,88 citrate de magnésium anhydre de 35,60 à 38,47 saccharose pour la compression directe, de 0,27 à 0,41 silice colloïdale anhydre, de 2,35 à 2,67 cyclamate de sodium, de 0,20 à 0,29 saccharine sodique et de 1,67 à 2,94 arômes.

2. Composition effervescente comprenant du monohydrate de métamizole sodique selon la prétention 1, **caractérisée en ce qu'**elle se présente sous la forme d'une poudre effervescente.

3. Composition effervescente comprenant du métamizole sodique monohydraté selon la prétention 1, **caractérisée en ce qu'**elle se présente sous la forme de granulés effervescents.

4. Procédé de préparation d'une composition effervescente selon les prétentions 1 et 3, **caractérisé en ce que** le métamizole sodique monohydraté est mélangé avec du carbonate de sodium anhydre et une partie de la silice colloïdale anhydre, le mélange sec est introduit pour la granulation par compactage au rouleau, les granulés obtenus. sont mélangés avec de l'acide citrique anhydre, du citrate de magnésium anhydre, du cyclamate de sodium, de la saccharine sodique, un arôme et la quantité restante de silice colloïdale anhydre, et le mélange granulaire résultant est tamisé et dosé dans un sachet de feuille à trois couches.
